# EUROPÄISCHE PATENTSCHRIFT

(11) **EP 0 770 401 B1**
(45) Veröffentlichungstag und Bekanntmachung des Hinweises auf die Patenterteilung: **20.11.2002**
(21) Anmeldenummer: 96115002.6
(22) Anmeldetag: 19.09.1996
(51) Int. Cl.: A61L 27/00, A61F 2/06, B29C 41/08, B29C 41/14, B29C 41/22, A61L 31/00

(54) **Verfahren zur Herstellung intraluminaler Stents aus bioresorbierbarem Polymermaterial**
Method of manufacturing intraluminal stents made of bioresorbable polymer material
Procédé de fabrication de stents intraluminaux en polymère biorésorbable

(30) Priorität: 24.10.1995 DE 19539449
(43) Veröffentlichungstag der Anmeldung: 02.05.1997
(73) Patentinhaber: BIOTRONIK Mess- und Therapiegeräte GmbH & Co Ingenieurbüro Berlin, 12359 Berlin (DE)
(72) Erfinder: Schmitz, Klaus-Peter, Prof. Dr., 18119 Warnemünde (DE); Behrend, Detlef, Dr., 18119 Warnemünde (DE)
(74) Vertreter: Hübner, Gerd, Dipl.-Phys.

(56) Entgegenhaltungen:
- EP-A- 0 332 371
- WO-A-91/17789
- WO-A-95/10989
- US-A- 4 409 172
- US-A- 4 955 899

## Beschreibung

Die Erfindung betrifft ein Verfahren zur Herstellung intraluminaler Stents aus bioresorbierbarem Polymermaterial.

Zum technologischen Hintergrund der Erfindung ist auszuführen, daß zur Herstellung z.B. implantierbarer intravaskulärer Stents - also von angioplastischen Gefäßwandstützen zum Einsatz in der Herzchirurgie - übliche kunststofftechnische Verfahren bekannt und angewendet werden, die auf der Verarbeitung thermoplastischer Polymermaterialien beruhen. So werden Stents, bei denen es sich um Röhrchen von einigen wenigen Millimetern Durchmesser und wenigen Zentimetern Länge handelt, durch Extrudieren oder Spritzgießen hergestellt. In diesem Zusammenhang ist auf den Fachaufsatz "Evaluation of poly(L-lactic acid) as a material for intravascular polymeric stents" von Agrawal et.al in Biomaterials 1992, Vol. 13, No. 3, S. 176 bis 187 zu verweisen, wo zur Herstellung von intravaskulären Polymerstents Poly(l-Milchsäure)-Monofilamente eingesetzt werden. Diese Monofilamente werden extrudiert und mit verschiedenen Streckraten gestreckt. Derart behandelte Monofilamente werden dann zur Konstruktion von Stents eingesetzt.

Der Nachteil der bekannten Verfahren besteht darin, daß sie zu einer thermischen Schädigung des Polymermaterials während des Extrusionsverfahrens oder des Spritzgießens führen können. Aufgrund der hohen Kristallinität der verwendeten Werkstoffe können zudem thermoplastische Umformverfahren sehr erschwert werden.

Aus dem Fachaufsatz "Fabrication of Resorbable Microporous Intravascular Stents for Gene Therapy Applications" von Rajasubramanian et al aus dem ASAIO Journal 1994, S. M584 bis M589 ist es ferner bekannt, resorbierbare, mikroporöse Stents aus einer Mischung von Poly-L-Milchsäure (PLLA) und Poly-E-Caprolacton (PCL) herzustellen, wobei sowohl spiral- als auch röhrenförmige Stent-Konstruktionen durch Lösen dieser Polymer-Mischung in einem organischen Lösungsmittel (1,4-Dioxan) und anschließende Schwimmaufbereitung des Polymers hergestellt werden. Bei letzterer wird die Polymerlösung auf eine gleichmäßig strömende Wasseroberfläche aufgesprüht, wodurch das Lösungsmittel im Wasser dispergiert und von der Oberfläche abdampft. Dadurch wird eine Polymerausfällung gebildet, die als Film auf der Wasseroberfläche schwimmt und an geeigneter Stelle von einem rotierenden Dorn aufgenommen wird, auf dem dadurch eine mehrlagige Beschichtung des teilweise ausgehärteten Polymers gebildet wird. Nach Fertigstellung der kompletten Beschichtung wird der Dorn in einen Vakuumofen gegeben, um für einen Zeitraum von 24 Stunden bei 45°C den Aushärtprozeß zu vollenden. Anschließend wird der Dorn mit der Polymerbeschichtung in 50 %-iger Ethanollösung eingeweicht, um die Polymerbeschichtung quellen zu lassen und sie anschließend vom Dorn abziehen zu können.

Das bekannte Verfahren ist insofern problematisch, als die Schwimmaufbereitung des Polymerfilms und das "Aufwickeln" des Polymerfilms auf den Dorn sehr diffizil und anfällig gegen Prozeßschwankungen sind. Darüber hinaus bedarf es einer aufwendigen Nachbehandlung der derart hergestellten Stent-Rohlinge. Ein weiterer Nachteil dieses Verfahrens liegt darin, daß sich mit dieser Herstellungstechnologie kein isotropes Gefüge im Stent erzielen läßt. Dies wirkt sich nachteilig für eine gleichmäßige Resorption in vivo und auf die mechanischen Eigenschaften des Stents aus.

In diesem Zusammenhang ist auch auf die US-A 4 409 172 hinzuweisen, die explizit die Herstellung einer vielschichtigen Röhre zur Verwendung im menschlichen Körper lehrt. Dabei ist ein in eine Lösung hängender Stab vorgesehen, der aus der Lösung unter Bilden einer Schicht herausgezogen wird. Diese trocknet, wonach das Gebilde in eine andersartige Lösung unter Wiederholung des Vorganges eingetaucht wird.

Auch WO-A-9510989 offenbart eine vielschichtige Vaskulärprothese, bei der Therapeutika in den einzelnen Lagen konzentriert und definiert enthalten sind. Insbesondere ist zwischen einer inneren porösen Traglage und einer äußeren Traglage eine quellbare Lage angeordnet, die ein Pharmazeutikum enthält und entsprechend freisetzen kann. Der aus diesem Dokument bekannte mehrlagige Stent wird durch Tauchen eines Kerns in unterschiedliche Lösungen hergestellt.

Die EP-A 0 332 371 offenbart ein Verfahren zur Bildung hohler Elastomerkörper mit poröser Oberfläche. Die Porosität wird durch Herauslösen von löslichen Granulaten aus einer Beschichtungslage hergestellt.

Die US-A- 4 955 899 schließlich bezieht sich auf einen vaskulären Stent, der aus einem anfänglich porösen PTFE-Röhrchen durch Komprimieren und Beschichten mit einem biokompatiblen Elastomer geschaffen wird.

Den aus dem Stand der Technik bekannten vaskulären Implantaten ist gemeinsam, daß sie kein isotropes Gefüge in ihrem Materialaufbau aufweisen, was in der erörterten Weise nachteilig ist.

Davon ausgehend liegt der Erfindung die Aufgabe zugrunde, ein Verfahren zur Herstellung intraluminaler Stents aus bioresorbierbaren Polymermaterial anzugeben, das unter prozeßtechnischer Vereinfachung zu Stents mit guten Bioresorptionseigenschaften führt.

Diese Aufgabe wird durch die im Anspruch 1 angegebenen Verfahrensschritte gelöst, die umfassen:
- Ansetzen einer viskosen Lösung von Poly-β-Hydroxybuttersäure als bioresorbierbares Polymermaterial in einem Lösungsmittel,
- sukzessives, schichtweises Aufbringen der viskosen Polymerlösung auf einen Positivformkern in mehreren Schritten durch Abscheiden des Polymermaterials unter Abdampfen des Lösungsmittels und unter zumindest teilweiser Auflösung der vorher abgeschiedenen Schicht zum Aufbau eines homogenen Stentrohlings,
- Abziehen des Stentrohlings vom Positivformkern, und
- Nachbearbeitung des Stentrohlings zur Endformgebung des Stents.

In vorteilhafter Weise wird bei dem erfindungsgemäßen Verfahren also nicht mehr von einer Polymermischung, sondern von einem einheitlichen Polymermaterial in Form von Poly-β-Hydroxybuttersäure ausgegangen. Ferner entfällt gegenüber dem Stand der Technik die aufwendige Schwimmaufbereitung zum Ausfällen des Polymerfilmes. Vielmehr wird - wie gemäß dem bevorzugten Ausführungsformen des erfindungsgemäßen Verfahrens nach den Ansprüchen 2 oder 3 vorgesehen ist - entweder mit einem mehrmaligen Tauchen des Positivformkerns in die Polymerlösung und anschließendem Entnehmen des Kerns aus der Lösung bzw. mit einem mehrmaligen, nacheinander stattfindenden Übergießen des Formkerns mit der Polymerlösung gearbeitet. Beide Alternativen sind prozeßtechnisch weit einfacher zu beherrschen und weniger anfällig gegen Prozeßfluktuationen, wie das oben geschilderte, aus dem Stand der Technik bekannte Verfahren.

Weiterhin haben sich Stents aus Poly-β-Hydroxybuttersäure als biologisch verträglich und gut bioresorbierbar erwiesen. Insofern dürfte die Anwendung der erfindungsgemäßen Stents auch nicht auf intravasale Stents beschränkt sein. Genausogut können Gefäßwandstützen für die Gastro- oder urologische Chirurgie hergestellt werden.

Durch die nach Anspruch 4 vorgesehene Rotation des Positivformkerns während des Aufbauvorganges des Stentrohlings wird dessen Homogenität durch eine Vergleichmäßigung des Aufbauvorganges verbessert.

Laut Anspruch 5 wird zum Ansetzen der Polymerlösung Poly-β-Hydroxybuttersäure als Pulver mit einem Anteil von 2 bis 3 Gew.% bezogen auf die Gesamtlösungsmenge in Chloroform gelöst. Dabei wird vorzugsweise in einem Temperaturbereich zwischen 50 und 70°C unter Rühren gearbeitet (Anspruch 6).

Nach Anspruch 7 ist vorgesehen, der Polymerlösung einen biokompatiblen Weichmacher zuzugeben, womit die mechanischen Eigenschaften des Stents entsprechend eingestellt werden. Als Weichmacher ist vorzugsweise Ethylcitrat in einer Konzentration von 5 bis 50 Gew.% bezogen auf die Gesamtlösungsmenge vorgesehen.

Ein besonderer verfahrenstechnischer Kniff ist in den Ansprüchen 8 bzw. 9 angegeben. Ganz allgemein wird demnach zur Verbesserung der Entformbarkeit der Stentrohlinge mit einem biokompatiblen Trennmittel gearbeitet, mit dem der Positivformkern vor dem Aufbringen der Polymerlösung beschichtet wird. Dieses Trennmittel ist unlöslich im Lösungsmittel der Polymerlösung, in einem anderen Lösungsmittel jedoch löslich. Insbesondere wird dabei als Trennmittel eine konzentrierte Zuckerlösung (Glukose oder Glukosidlösungen) verwendet, die durch destilliertes Wasser vor dem Abziehen des Stentrohlings vom Positivformkern herausgelöst wird. Da solche Zuckerlösungen in destilliertem Wasser sehr gut, in Chloroform jedoch nicht löslich sind, stellt die daraus hergestellte Beschichtung des Positivformkerns einen guten Aufbauuntergrund beim Aufbringen der viskosen Polymerlösung dar. Nach Fertigstellung des Stentrohlings wird die Beschichtung durch das destillierte Wasser sehr schnell herausgelöst, wodurch zwischen Positivformkern und dem empfindlichen Stentrohling ein Ringspalt entsteht, folglich der empfindliche Rohling praktisch lose auf dem Formkern sitzt und einfach abgezogen werden kann.

Die Ansprüche 10 und 11 kennzeichnen Verfahrensmaßnahmen, mit denen die Produkteigenschaften des damit hergestellten Stents verbessert werden. So können zur kontinuierlichen Freisetzung von gerinnungs- oder zellproliferationshemmenden Pharmaka bei der Bioresorption des Stents diese Mittel in sein Volumen eingebaut werden, indem diese Pharmaka in die Polymerlösung zugegeben werden. Die Verteilung der Pharmaka kann dabei homogen über das Stentvolumen oder in Schichten sein. In letzterem Falle wird bei den einzelnen Tauch- oder Übergießvorgängen mit unterschiedlichen Polymerlösungen (mit bzw. ohne zugesetzte Pharmaka) gearbeitet. In analoger Weise können nach Anspruch 11 Silber oder Silberverbindungen der Polymerlösung zugesetzt werden, um als Röntgenkontrastmittel und/oder entzündungshemmendes Mittel homogen oder in Schichten verteilt in das Stentvolumen eingebaut zu werden.

Der nach Anspruch 12 vorgesehene Filtrierschritt für die Polymerlösung dient deren Homogenisierung und Reinigung.

Als vorteilhafte mechanische Bearbeitungsmethode zur Nachbearbeitung der Stentrohlinge hat sich das Laser- oder Wasserstrahlschneiden herauskristallisiert (Anspruch 13).

Weitere Merkmale, Einzelheiten und Vorteile der Erfindung sind der nachfolgenden Beschreibung entnehmbar, in der ein Ausführungsbeispiel des erfindungsgemäßen Verfahrens unter Bezugnahme auf die beigefügten Zeichnungen näher erläutert wird. Es zeigen
- Fig. 1: eine schematische perspektivische Darstellung eines auf einen Positivformkern aufgebauten Stentrohlings, und
- Fig. 2: einen ausschnittsweisen, vergrößerten Querschnitt durch den auf dem Formkern sitzenden Stentrohling gemäß Fig. 1.

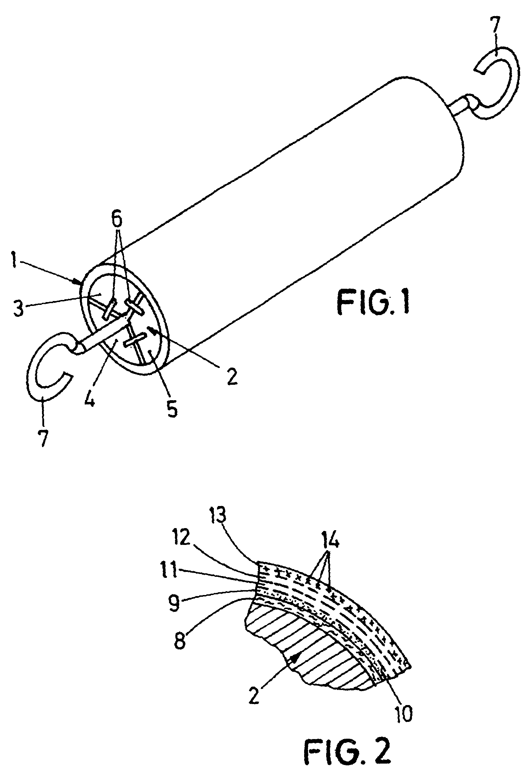

Ein erfindungsgemäßes Verfahren zur Herstellung eines resorbierbaren intravasalen Stents wird unter Bezugnahme auf die Fig. 1 und 2 im folgenden näher erörtert.

Es werden im vorliegenden Ausführungsbeispiel drei Polymerlösungen angesetzt, die hinsichtlich der Art und Menge des Polymers und des Lösungsmittels übereinstimmen. Dazu werden jeweils 4 g pulverförmige Poly-β-Hydroxybuttersäure in 100 ml Chloroform als organischem Lösungsmittel unter Rühren mit einem Magnetrührer bei einer Temperatur von 56°C gelöst. Weiterhin wird der Lösung ein biokompatibler Weichmacher, nämlich Ethylcitrat in einer Konzentration von 5 bis 50 Gew.% zur Einstellung der gewünschten mechanischen Eigenschaften des Stents der Lösung unter Rühren beigefügt.

Der ersten der drei Lösungen wird zusätzlich ein gerinnungshemmendes pharmazeutisches Mittel zugesetzt. Der dritten der drei Lösungen werden Silberteilchen beigemischt, die in fertigen Stent als Röntgenkontrastmittel dienen. Die Lösungen werden anschließend durch eine Fritte vorzugsweise des Typs 3g3 filtriert.

Zur Ausformung eines Stentrohlings 1, wie er in Fig. 1 und ausschnittsweise in Fig. 2 gezeigt ist, dient ein sogenannter Positivformkern 2, bei dem es sich im wesentlichen um ein Zylinderteil mit mehreren Zentimeter Länge und einigen wenigen Millimeter Durchmesser handelt. Wie aus Fig. 1 deutlich wird ist der Positivformkern 2 aus drei im Querschnitt sektorförmigen Elementen 3, 4, 5 zusammengesetzt, die mit Hilfe von Verkeilungselementen 6 zusammengehalten werden. An seinen stirnseitigen Enden ist der Positivformkern 2 ferner mit Befestigungsösen 7 versehen, mit deren Hilfe der Positivformkern 2 gehandhabt werden kann. Das für dem Positivformkern 2 verwendete Material ist ein nicht-adhäsiver Werkstoff, wie z.B. Polytetrafluorethylen.

Wie in Fig. 2 angedeutet ist, wird der Positivformkern 2 vor dem Aufbringen des Stentrohlings 1 mit einer biokompatiblen Trennmittelbeschichtung 8 aus einer konzentrierten Glukosidlösung versehen, die in Chloroform nicht, in destilliertem Wasser jedoch sehr gut löslich ist.

Zum sukzessiven, schichtweisen Aufbau des Stentrohlings 1 wird nun der so vorbereitete Positivformkern 2 in die erste angesetzte Lösung eingetaucht und nach Abwarten einer kurzen Zeitspanne wieder daraus entnommen. Es bleibt eine erste Schicht 9 dieser Polymerlösung auf der Trennmittelbeschichtung 8 haften. Unter Rotation des Positivkerns 2 wird eine kurze Zeitspanne abgewartet, in der die Poly-β-Hydroxybuttersäure unter Abdampfen des Chloroforms abgeschieden wird. Wie in Fig. 2 durch eine Punktierung angedeutet ist, ist in dieser ersten Schicht 9 das gerinnungshemmende pharmazeutische Mittel 10 homogen verteilt.

Anschließend wird der Formkern 2 in die zweite Polymerlösung eingetaucht, in der weder gerinnungshemmende Mittel, noch Silberteilchen vorhanden sind. Nach kurzer Zeit wird der Formkern 2 wieder aus dieser Lösung entnommen, wodurch die zweite Schicht 11 auf der ersten Schicht 9 haften bleibt. Unter Rotation des Formkerns 2 findet wiederum ein Abscheiden der Poly-β-Hydroxybuttersäure unter Abdampfen von Chloroform statt. Dabei wird die erste Schicht 9 zumindest teilweise angelöst und somit auch eine innige molekulare Verbindung zur ersten Schicht 9 hergestellt, so daß der Stentrohling 1 hinsichtlich seiner Polymerstruktur homogen ist.

Der Vorgang wird zur Bildung der dritten Schicht 12 wiederholt.

Zur Bildung der vierten Schicht 13, die die Außenschicht des Stentrohlings 1 darstellt, wird der Formkern 2 in die dritte Lösung eingetaucht, in der Silberteilchen verteilt sind. Nach der Entnahme des Formkerns 2 aus dieser Lösung bleibt diese vierte Schicht 13 auf der dritten Schicht 12 haften, wobei in der vierten Schicht 13 - wie in Fig. 2 durch Kreuze angedeutet - Silberteilchen 14 homogen verteilt sind. Diese dienen - wie erörtert - als Röntgenkontrastmittel.

Der Formkern mit dem darauf aufgebauten Stentrohling 1 wird anschließend über eine Zeitspanne von 2 bis 10 Minuten unter Rotation getrocknet, wobei alle Schichten 9, 11, 12, 13 vollständig auspolymerisieren und in sich sowie untereinander eine innige molekulare Verbindung eingehen, so daß hinsichtlich der Polymerstruktur im fertig auspolymerisierten Stentrohling 1 keine Inhomogenitäten - also keine Schichtstruktur - mehr festzustellen sind. Zur Innenseite hin sind lediglich die gerinnungshemmenden Pharmaka 10 und zur Außenseite hin die Silberteilchen 14 feststellbar.

Zum Abziehen des Stentrohlings 1 vom Positivformkern 2 wird die gesamte Anordnung in destilliertes Wasser getaucht, das die Trennmittelbeschichtung 8 herauslöst. Dadurch sitzt der Stentrohling 1 lose auf dem Positivformkern 2 und kann praktisch widerstandsfrei abgezogen werden.

Der abgezogene Stentrohling 1 wird anschließend durch Laserschneiden auf seine Soll-Länge geschnitten, die in der Regel etwa 3 cm beträgt. Sein Außendurchmesser beträgt etwa 3 mm, seine Wanddicke einige Zehntel Millimeter. Insofern versteht sich, daß die Darstellung in Fig. 2 lediglich schematischer Natur ist und die Schichtdicken stark übertrieben zeigt.

Bei der Implantation eines derartigen Stents kann seine Lage in einem Herzkranzgefäß aufgrund der Silberteilchen 14 in der Außenschicht röntgenographisch gut überprüft werden. Ferner fördern die gerinnungshemmenden Pharmaka 10 in der innenliegenden ersten Schicht 9 die Bioverträglichkeit des implantierten Stents. Sie werden bei dessen Bioresorption kontinuierlich freigesetzt, so daß sich eine Langzeitwirkung ergibt.

Abschließend ist darauf hinzuweisen, daß die gerinnungshemmenden Pharmaka 10 und die Silberteilchen 14 nicht nur schichtweise, sondern auch homogen im Stentrohling-Volumen verteilt eingebaut werden können. In diesem Falle wird nur mit einer einzigen Polymerlösung gearbeitet, die auch die gerinnungshemmenden Pharmaka und Silberteilchen enthält.

## Patentansprüche

1. Verfahren zur Herstellung intravasaler Stents aus bioresorbierbarem Polymermaterial mit folgenden Verfahrensschritten:
- Ansetzen einer viskosen Lösung von Poly-β-Hydroxybuttersäure als bioresorbierbares Polymermaterial in einem Lösungsmittel,
- sukzessives, schichtweises Aufbringen der Polymerlösung auf einen Positivformkern (2) in mehreren Schritten durch Abscheiden des Polymermaterials unter Abdampfen des Lösungsmittels und unter zumindest teilweiser Anlösung der vorher abgeschiedenen Schicht zum Aufbau eines in seiner Polymerstruktur homogenen Stentrohlings (1),
- Abziehen des Stentrohlings (1) vom Positivformkern (2) und
- Nachbearbeitung des Stentrohlings (1) zur Endformgebung des Stents.

2. Verfahren nach Anspruch 1, **dadurch gekennzeichnet, daß** zum sukzessiven, schichtweisen Aufbringen der Polymerlösung der Positivformkern (2) mehrmals abwechselnd in die Polymerlösung eingetaucht und zum Abdampfen des Lösungsmittels aus der sich darauf anlagernden Polymerlösungsschicht (9, 11, 12, 13) aus der Polymerlösung entnommen wird.

3. Verfahren nach Anspruch 1, **dadurch gekennzeichnet, daß** zum sukzessiven, schichtweisen Aufbringen der Polymerlösung der Positivformkern (2) mehrmals nacheinander mit der Polymerlösung übergossen wird, wobei zwischen jedem Übergießen die vorher anhaftende Polymerlösungsschicht (9, 11, 12, 13) von der nachfolgenden Polymerlösungsschicht (11, 12, 13) zumindest teilweise angelöst und das Polymermaterial durch Abdampfen des Lösungsmittels abgeschieden wird.

4. Verfahren nach einem der Ansprüche 1 bis 3, **dadurch gekennzeichnet, daß** der Positivformkern (2) während des Aufbauvorganges des Stentrohlings (1) in Rotation versetzt wird.

5. Verfahren nach einem der Ansprüche 1 bis 4, **dadurch gekennzeichnet, daß** zum Ansetzen der Polymerlösung Poly-β-Hydroxybuttersäure als Pulver mit einem Anteil von 2 bis 3 Gew.%, vorzugsweise etwa 2,5 Gew.% bezogen auf die Gesamtlösungsmenge in Chloroform gelöst wird.

6. Verfahren nach Anspruch 5, **dadurch gekennzeichnet, daß** das Lösen unter Rühren in einem Temperaturbereich zwischen 50 und 70°C, vorzugsweise bei 56°C stattfindet.

7. Verfahren nach einem der Ansprüche 1 bis 6, **dadurch gekennzeichnet, daß** der Polymerlösung eine biokompatibler Weichmacher, vorzugsweise Ethylcitrat in einer Konzentration von 5 bis 50 Gew.%, zur Einstellung der mechanischen Eigenschaften des Stents zugefügt wird.

8. Verfahren nach einem der Ansprüche 1 bis 7, **dadurch gekennzeichnet, daß** der Positivformkern (2) vor dem Aufbringen der Polymerlösung mit einem biokompatiblen Trennmittel beschichtet wird (Trennmittelbeschichtung 8), das in Chloroform unlöslich, in einem anderen Lösungsmittel jedoch löslich ist.

9. Verfahren nach Anspruch 8, **dadurch gekennzeichnet, daß** als Trennmittel eine konzentrierte Zuckerlösung verwendet wird, die durch destilliertes Wasser vor dem Abziehen des Stentrohlings (1) vom Positivformkern (2) herausgelöst wird.

10. Verfahren nach einem der Ansprüche 1 bis 9, **dadurch gekennzeichnet, daß** durch Zugabe von gerinnungs- oder zellproliferationshemmenden Pharmaka (10) in die Polymerlösung diese Pharmaka (10) zur kontinuierlichen Freisetzung bei der Bioresorption des Stents in dessen Volumen homogen oder in eine oder mehrere Schichten (9) verteilt eingebaut werden.

11. Verfahren nach einem der Ansprüche 1 bis 10, **dadurch gekennzeichnet, daß** Teilchen (14) von Silber oder Silberverbindungen in die Polymerlösung zugegeben werden, um als Röntgenkontrastmittel und/oder entzündungshemmendes Mittel homogen oder in eine oder mehrere Schichten (13) verteilt in das Stent-Volumen eingebaut zu werden.

12. Verfahren nach einem der Ansprüche 1 bis 11, **dadurch gekennzeichnet daß** die Polymerlösung vor dem Aufbringen auf den Positivformkern (2) durch eine Fritte filtriert wird.

13. Verfahren nach einem der Ansprüche 1 bis 12, **dadurch gekennzeichnet daß** der Stentrohling (1) zur Nachbearbeitung mittels Laser- oder Wasserstrahlschneiden bearbeitet wird.

## Claims

1. A method for the manufacture of intravascular stents of bioresorbable polymeric material, comprising the following steps:
- preparing a viscous solution of poly-β-hydroxybutanoic acid as a bioresorbable polymeric material in a solvent,
- successively coating a male mold core (2) with layers of the polymer solution in several steps by precipitation of the polymeric material by the solvent being evaporated and by the layer previously precipitated being dissolved at least partially for a homogeneous stent blank (1) to build up,
- drawing the stent blank (1) off the male mold core (2), and
- subsequently treating the stent blank (1) to finish the shaping of the stent.

2. A method according to claim 1, **characterized in that** for successively coating the male mold core (2) with layers of the polymer solution, the male mold core (2) is alternatively dipped for several times into the polymer solution and removed from the latter for the solvent to evaporate from the layer (9, 11, 12, 13) of polymer solution depositing.

3. A method according to claim 1, **characterized in that** for successively coating the male mold core (2) with layers of the polymer solution, the latter is poured over the male mold core (2) in several succesive operations, the previously adhering layer (9, 11, 12, 13) of polymer solution, between each pouring operation, being at least partially dissolved by the subsequent layer (11, 12, 13) of polymer solution and the polymeric material being precipitated by evaporation of the solvent.

4. A method according to one of claims 1 to 3, **characterized in that** the male mold core (2) is rotated during the build-up of the stent blank (1).

5. A method according to one of claims 1 to 4, **characterized in that** preparing the polymer solution consists in dissolving in chloroform poly-β-hydroxybutanoic acid as a powder in a percentage of 2 to 3 % by weight, preferably about 2.5 % by weight, referred to the total quantity of solution.

6. A method according to claim 5, **characterized in that** dissolving takes place by stirring at temperatures ranging between 50 and 70°C, preferably at 56°C.

7. A method according to one of claims 1 to 6, **characterized in that** for adjustment of the mechanical properties of the stent, a biocompatible plasticizer, preferably ethyl citrate of a concentration of 5 to 50 % by weight, is added to the polymer solution.

8. A method according to one of claims 1 to 7, **characterized in that** prior to being coated with the polymer solution, the male mold core (2) is coated with a biocompatible release agent (release coating 8), which is insoluble in chloroform, but soluble in other solvents.

9. A method according to claim 8, **characterized in that** the release agent used is a concentrated sugar solution, which is dissolved by distilled water before the stent blank ( I ) is drawn off the male mold core (2).

10. A method according to one of claims 1 to 9, **characterized in that** by the addition of pharmaceutical anticoagulant or cell-proliferation-inhibiting agents (10) to the polymer solution, these agents (10) are incorporated in the volume of the stent homogeneously or spread in one or several layers (9) for being continuously released during bioresorption of the stent.

11. A method according to one of claims 1 to 10, **characterized in that** particles (14) of silver or silver alloys are added to the polymer solution for being incorporated as an X-ray contrast medium and/or inflammation-inhibiting agent into the volume of the stent homogeneously or spread in one or several layers (13).

12. A method according to one of claims 1 to 11, **characterized in that** prior to the coating of the male mold core (2) with the polymer solution, the latter is filtered through a frit.

13. A method according to one of claims 1 to 12, **characterized in that** the stent blank (1) is subsequently treated by laser-beam or water-jet cutting.

## Revendications

1. Procédé pour la production de stents intravasculaires en polymère biorésorbable selon les étapes de procédé suivantes :
- préparation d'une solution visqueuse de poly(acide □-hydroxybutyrique) comme polymère biorésorbable dans un solvant,
- application successive par couche de la solution polymère sur un noyau de moule positif (2) en plusieurs étapes par dépôt de la matière polymère en faisant évaporer le solvant et en corrodant au moins partiellement la couche précédemment déposée pour la construction d'une ébauche de stent (1) homogène dans sa structure polymère,
- démoulage de l'ébauche de stent (1) du noyau de moule positif (2) et
- refaçonnage de l'ébauche de stent (1) pour donner la forme de finition au stent.

2. Procédé selon la revendication 1, **caractérisé en ce que** pour l'application successive par couche de la solution polymère, le noyau de moule positif (2) est plongé plusieurs fois alternativement dans la solution polymère, et qu'il est retiré de la solution polymère afin d'évaporer le solvant de la couche de solution polymère (9, 11, 12, 13) fixée dessus.

3. Procédé selon la revendication 1, **caractérisé en ce que** pour l'application successive par couche de la solution polymère, le noyau de moule positif (2) est aspergé plusieurs fois en succession avec la solution polymère, la couche de solution polymère (9, 11, 12, 13) précédemment adhérente étant au moins partiellement corrodée par la couche de solution polymère suivante (11, 12, 13) entre chaque aspersion, et la matière polymère étant déposée par évaporation du solvant.

4. Procédé selon l'une quelconque des revendications 1 à 3, **caractérisé en ce que** le noyau de moule positif (2) est déplacé en rotation pendant le processus de construction de l'ébauche de stent (1).

5. Procédé selon l'une quelconque des revendications 1 à 4, **caractérisé en ce que** pour la préparation de la solution polymère, du poly(acide □-hydroxybutyrique) est dissous sous forme de poudre dans du chloroforme avec une proportion de 2 à 3% en poids, de préférence environ 2,5% en poids par rapport à la quantité totale de la solution.

6. Procédé selon la revendication 5, **caractérisé en ce que** la dissolution a lieu par brassage dans une plage de températures comprises entre 50 et 70°C, de préférence à 56°C.

7. Procédé selon l'une quelconque des revendications 1 à 6, **caractérisé en ce qu'**un plastifiant biocompatible, de préférence le citrate d'éthyle est ajouté à la solution polymère, en une concentration de 5 à 50% en poids, pour ajuster les propriétés mécaniques du stent.

8. Procédé selon l'une quelconque des revendications 1 à 7, **caractérisé en ce que** le noyau de moule positif (2), avant l'application de la solution polymère, est revêtu d'un agent séparateur biocompatible (revêtement d'agent séparateur 8) insoluble dans le chloroforme, toutefois soluble dans un autre solvant.

9. Procédé selon la revendication 8, **caractérisé en ce qu'**une solution sucrée concentrée est employée comme agent séparateur, qui est éliminée par de l'eau distillée avant le démoulage de l'ébauche de stent (1) du noyau de moule positif (2).

10. Procédé selon l'une quelconque des revendications 1 à 9, **caractérisé en ce que** par l'addition de produits pharmaceutiques (10) anticoagulants ou inhibant la prolifération cellulaire dans la solution polymère, ces produits pharmaceutiques (10) sont incorporés de manière homogène ou répartie en plusieurs couches pour être libérés en continu lors de la biorésorption du stent dans son volume (9).

11. Procédé selon l'une quelconque des revendications 1 à 10, **caractérisé en ce que** des particules (14) d'argent ou de composés d'argent sont ajoutées à la solution polymère afin d'être incorporées de manière homogène sous forme d'agents de contraste radioscopique et/ou d'agents anti-inflammatoires ou de façon répartie en plusieurs couches dans le volume de stent.

12. Procédé selon l'une quelconque des revendications 1 à 11, **caractérisé en ce que** la solution polymère est filtrée à travers une fritte avant l'application sur le noyau de moule positif (2).

13. Procédé selon l'une quelconque des revendications 1 à 12, **caractérisé en ce que** l'ébauche de stent (1) pour le refaçonnage est façonnée par découpage au laser ou au jet d'eau.
